# EUROPEAN PATENT SPECIFICATION

(11) **EP 2 235 093 B1**
(45) Date of publication and mention of the grant of the patent: **22.04.2015**
(21) Application number: 08863486.0
(22) Date of filing: 16.12.2008
(51) Int. Cl.: C08J 3/24, A61L 15/60, C08F 8/44, C08L 101/14, C08K 5/00

(54) **PROCESS FOR PRODUCING SURFACE-CROSSLINKED SUPERABSORBENTS**
VERFAHREN ZUR HERSTELLUNG VON OBERFLÄCHENVERNETZTEN SUPERABSORBERN
PROCÉDÉ POUR PRODUIRE DES SUPERABSORBANTS RÉTICULÉS EN SURFACE

(30) Priority: 19.12.2007 US 14840 P
(43) Date of publication of application: 06.10.2010
(73) Proprietor: BASF SE, 67056 Ludwigshafen (DE)
(72) Inventor: HERFERT, Norbert, 63674 Altenstadt (DE); STUEVEN, Uwe, 65812 Bad Soden (DE); CHIANG, William, G-J, Charlotte NC 28226 (US)
(86) International application number: PCT/EP2008/067614
(87) International publication number: WO 2009/080611

(56) References cited:
- EP-A2- 1 516 884
- WO-A1-2007/037522
- WO-A2-01/89591
- WO-A2-2007/121937

## Description

The present invention relates to a process for producing surface-crosslinked superabsorbents that exhibit superior permeability and absorption properties. In particular, the present invention relates to a process for producing superabsorbents wherein the superabsorbent particles are coated with a permeability enhancing agent.

Superabsorbents are known. Superabsorbents are materials that are able to take up and retain several times their weight in water, possibly up to several hundred times their weight, even under moderate pressure. Absorbing capacity is usually lower for salt-containing solutions compared to distilled or otherwise de-ionised water. Typically, a superabsorbent has a centrifugal retention capacity ("CRC", method of measurement see hereinbelow) of at least 5 g/g, preferably at least 10 g/g and more preferably at least 15 g/g. Such materials are also commonly known by designations such as "high-swellability polymer", "hydrogel" (often even used for the dry form), "hydrogel-forming polymer", "water-absorbing polymer", "absorbent gel-forming material", "swellable resin", "water-absorbing resin" or the like. The materials in question are crosslinked hydrophilic polymers, in particular polymers formed from (co)polymerized hydrophilic monomers, graft (co)polymers of one or more hydrophilic monomers on a suitable grafting base, crosslinked ethers of cellulose or starch, crosslinked carboxymethylcellulose, partially crosslinked polyalkylene oxide or natural products that are swellable in aqueous fluids, examples being guar derivatives, of which water-absorbing polymers based on partially neutralized acrylic acid are most widely used. Superabsorbents are usually produced, stored, transported and processed in the form of dry powders of polymer particles, "dry" usually meaning less than 5 wt.-% water content. A superabsorbent transforms into a gel on taking up a liquid, specifically into a hydrogel when as usual taking up water. By far the most important field of use of superabsorbents is the absorbing of bodily fluids. Superabsorbents are used for example in diapers for infants, incontinence products for adults or feminine hygiene products. Examples of other fields of use are as water-retaining agents in market gardening, as water stores for protection against fire, for liquid absorption in food packaging or, in general, for absorbing moisture.

Processes for producing superabsorbents are also known. The acrylate-based superabsorbents which dominate the market are produced by radical polymerization of acrylic acid in the presence of a crosslinking agent (the "internal crosslinker"), usually in the presence of water, the acrylic acid being neutralized to some degree in a neutralization step conducted prior to or after polymerization, or optionally partly prior to and partly after polymerization, usually by adding a alkali, most often an aqueous sodium hydroxide solution. This yields a polymer gel which is comminuted (depending on the type of reactor used, comminution may be conducted concurrently with polymerization) and dried. Usually, the dried powder thus produced (the "base polymer") is surface crosslinked (also termed surface "post"crosslinked) by adding further organic or polyvalent metal (i.e. cationic) crosslinkers to generate a surface layer which is crosslinked to a higher degree than the particle bulk. Most often, aluminium sulphate is being used as polyvalent metal crosslinker. Applying polyvalent metal cations to superabsorbent particles is sometimes not regarded as surface crosslinking, but termed "surface complexing" or as another form of surface treatment, although it has the same effect of increasing the number of bonds between individual polymer strands at the particle surface and thus increases gel particle stiffness as organic surface crosslinkers have. Organic and polyvalent metal surface crosslinkers can be cumulatively applied, jointly or in any sequence.

Surface crosslinking leads to a higher crosslinking density close to the surface of each superabsorbent particle. This addresses the problem of "gel blocking", which means that, with earlier types of superabsorbents, a liquid insult will cause swelling of the outermost layer of particles of a bulk of superabsorbent particles into a practically continuous gel layer, which effectively blocks transport of further amounts of liquid (such as a second insult) to unused superabsorbent below the gel layer. While this is a desired effect in some applications of superabsorbents (for example sealing underwater cables), it leads to undesirable effects when occurring in personal hygiene products. Increasing the stiffness of individual gel particles by surface crosslinking leads to open channels between the individual gel particles within the gel layer and thus facilitates liquids transport through the gel layer. Although surface crosslinking decreases the CRC or other parameters describing the total absorption capacity of a superabsorbent sample, it may well increase the amount of liquid that can be absorbed by hygiene product containing a given amount of superabsorbent.

Other means of increasing the permeability (to be precise, the "gel bed permeability", "GBP" or the "saline flow conductivity", "SFC") of a superabsorbent are also known. These include admixing of superabsorbent with fibres such as fluff in a diaper core or admixing other components that increase gel stiffness or otherwise create open channels for liquid transportation in a gel layer. Usually, components increasing the GBP or SFC are referred to as permeability enhancing agents ("PEA").

Frederic L. Buchholz und Andrew T. Graham (Hrsg.) in: "Modern Superabsorbent Polymer Technology", J. Wiley & Sons, New York, U.S.A. / Wiley-VCH, Weinheim, Germany, 1997, ISBN 0-471-19411-5, give a comprehensive overview over superabsorbents and processes for producing superabsorbents. In its section on advanced products comprising surface crosslinking, aluminium acetate is disclosed as surface crosslinker.

WO 98/48 857 A1 discloses superabsorbent particles which are surface crosslinked by polyvalent metal cations such as Al, Fe, Zr, Mg and Zn ions and treated with a liquid binder such as water, mineral oil or polyols, preferably water. WO 01/74 913 A1 relates to regenerating the gel bed permeability of surface-crosslinked superabsorbents, in particular surface-crosslinked superabsorbents which have been subject to attrition, by adding a solution of a salt of an at least trivalent cation, typically an aqueous solution of aluminium sulphate.

US 6 620 889 B1 discloses superabsorbents which are surface crosslinked with a combination of a polyol and a cation salt in aqueous solution. The salt's anion may be chloride, bromide, sulphate, carbonate, nitrate, phosphate, acetate or lactate. Using aluminium sulphate is preferred. According to the teaching of WO 2006/111 402 A2, a base polymer is contacted with a permeability enhancing agent selected from silicon-oxygen-compounds, polyvalent, preferably trivalent cation salts or a mixture thereof. The trivalent cation salt is preferably an aluminium salt selected from a range that includes aluminium lactate, oxalate, citrate, glyoxylate, succinate, tartrate and other organic or inorganic aluminium salts. WO 2005/108 472 A1 discloses a process that includes treating a base polymer with a water-soluble multivalent metal salt and an organic acid or its salt. The multivalent metal salt is preferably aluminium sulphate. The organic acid or salt is selected from a range of acids that includes citric acid, glyoxylic acid, glutaric acid, succinic acid, tartaric acid and lactic acid, or alkali or ammonium salts thereof.

WO 2007/037522 A1 discloses a water-absorbent agent composition containing as a main component a polycarboxylic acid water-absorbent agent having a crosslinking structure. In a the provided examples a first surface crosslinking agent aqueous solution prepared by mixing propylene glycol, 1,4-butanediol and water was sprayed to and mixed with water absorbent agent. The resulting mixture was subjected to heat treatment at powder temperature of 195 °C for 45 min. After, second surface crosslinking agent aqueous solution prepared by mixing propylene glycol, aluminum sulfate, sodium lactate and water was sprayed to and mixed with the mixture. The resulting mixture was subjected to heating treatment at 60 °C for an hour to obtain a surface-crosslinked water absorbent agent.

It is an object of the present invention to provide a process for producing a superabsorbent which exhibits high gel-bed permeability.

We have found that this object is achieved by a process for producing a surface-crosslinked superabsorbent that comprises the steps of contacting a superabsorbent base polymer with an polyvalent metal salt solution produced by dissolving at least two different polyvalent metal salts, at least one of which comprises a chelate-forming anion, and heat-treating to produce a surface-crosslinked superabsorbent.

The superabsorbent in the present invention is a superabsorbent capable of absorbing and retaining amounts of water equivalent to many times its own weight under a certain pressure. In general, it has a centrifugal retention capacity (CRC, method of measurement see hereinbelow) of at least 5 g/g, preferably at least 10 g/g and more preferably at least 15 g/g. Preferably, the superabsorbent is a crosslinked polymer based on partially neutralized acrylic acid, and it is surface postcrosslinked. A "superabsorbent" can also be a mixture of chemically different individual superabsorbents in that it is not so much the chemical composition which matters as the superabsorbing properties.

Processes for producing superabsorbents, including surface-postcrosslinked superabsorbents, are known. Synthetic superabsorbents are obtained for example by polymerization of a monomer solution comprising
a) at least one ethylenically unsaturated acid-functional monomer,
b) at least one crosslinker (usually designated the "internal" crosslinker(s)),
c) optionally one or more ethylenically and/or allylically unsaturated monomers copolymerizable with the monomer a), and
d) optionally one or more water-soluble polymers onto which the monomers a), b) and if appropriate c) can be at least partly grafted.

Suitable monomers a) are for example ethylenically unsaturated carboxylic acids, such as acrylic acid, methacrylic acid, maleic acid, fumaric acid and itaconic acid, or derivatives thereof, such as acrylamide, methacrylamide, acrylic esters and methacrylic esters. Acrylic acid and methacrylic acid are particularly preferred monomers. Acrylic acid is most preferable.

The monomers a) and especially acrylic acid comprise preferably up to 0.025% by weight of a hydroquinone half ether. Preferred hydroquinone half ethers are hydroquinone monomethyl ether (MEHQ) and/or tocopherols such as alpha-tocopherol, especially racemic alpha-tocopherol or particularly RRR-alpha-Tocopherol.

The monomer solution comprises preferably not more than 130 weight ppm, more preferably not more than 70 weight ppm, preferably not less than 10 weight ppm, more preferably not less than 30 weight ppm and especially about 50 weight ppm of hydroquinone half ether, all based on acrylic acid, with acrylic acid salts being arithmetically counted as acrylic acid. For example, the monomer solution can be produced using an acrylic acid having an appropriate hydroquinone half ether content.

Crosslinkers b) are compounds having at least two polymerizable groups which can be free-radically interpolymerized into the polymer network. Useful crosslinkers b) include for example ethylene glycol dimethacrylate, diethylene glycol diacrylate, allyl methacrylate, trimethylolpropane triacrylate, triallylamine, tetraallyloxyethane as described in EP 530 438 A1, di- and triacrylates as described in EP 547 847 A1, EP 559 476 A1, EP 632 068 A1, WO 93/21 237 A1, WO 03/104 299 A1, WO 03/104 300 A1, WO 03/104 301 A1 and DE 103 31 450 A1, mixed acrylates which, as well as acrylate groups, comprise further ethylenically unsaturated groups, as described in DE 103 31 456 A1 and WO 04/013 064 A2, or crosslinker mixtures as described for example in DE 195 43 368 A1, DE 196 46 484 A1, WO 90/15 830 A1 and WO 02/032 962 A2.

Useful crosslinkers b) include in particular N,N'-methylenebisacrylamide and N,N'-methylenebismethacrylamide, esters of unsaturated mono- or polycarboxylic acids of polyols, such as diacrylate or triacrylate, for example butanediol diacrylate, butanediol dimethacrylate, ethylene glycol diacrylate, ethylene glycol dimethacrylate and also trimethylolpropane triacrylate and allyl compounds, such as allyl (meth)acrylate, triallyl cyanurate, diallyl maleate, polyallyl esters, tetraallyloxyethane, triallylamine, tetraallylethylenediamine, allyl esters of phosphoric acid and also vinylphosphonic acid derivatives as described for example in EP 343 427 A2. Useful crosslinkers b) further include pentaerythritol diallyl ether, pentaerythritol triallyl ether, pentaerythritol tetraallyl ether, polyethylene glycol diallyl ether, ethylene glycol diallyl ether, glycerol diallyl ether, glycerol triallyl ether, polyallyl ethers based on sorbitol, and also ethoxylated variants thereof. The process of the present invention may utilize di(meth)acrylates of polyethylene glycols, the polyethylene glycol used having a molecular weight between 300 and 1000.

However, particularly advantageous crosslinkers b) are di- and triacrylates of 3- to 15-tuply ethoxylated glycerol, of 3- to 15-tuply ethoxylated trimethylolpropane, of 3- to 15-tuply ethoxylated trimethylolethane, especially di- and triacrylates of 2- to 6-tuply ethoxylated glycerol or of 2- to 6-tuply ethoxylated trimethylolpropane, of 3-tuply propoxylated glycerol, of 3-tuply propoxylated trimethylolpropane, and also of 3-tuply mixedly ethoxylated or propoxylated glycerol, of 3-tuply mixedly ethoxylated or propoxylated trimethylolpropane, of 15-tuply ethoxylated glycerol, of 15-tuply ethoxylated trimethylolpropane, of 40-tuply ethoxylated glycerol, of 40-tuply ethoxylated trimethylolethane and also of 40-tuply ethoxylated trimethylolpropane.

Very particularly preferred for use as crosslinkers b) are diacrylated, dimethacrylated, triacrylated or trimethacrylated multiply ethoxylated and/or propoxylated glycerols as described for example in WO 03/104 301 A1. Di- and/or triacrylates of 3- to 10-tuply ethoxylated glycerol are particularly advantageous. Very particular preference is given to di- or triacrylates of 1- to 5-tuply ethoxylated and/or propoxylated glycerol. The triacrylates of 3- to 5-tuply ethoxylated and/or propoxylated glycerol are most preferred. These are notable for particularly low residual contents (typically below 10 weight ppm) in the water-absorbing polymer and the aqueous extracts of the water-absorbing polymers produced therewith have an almost unchanged surface tension (typically at least 0.068 N/m) compared with water at the same temperature.

Examples of ethylenically unsaturated monomers c) which are copolymerizable with the monomers a) are acrylamide, methacrylamide, crotonamide, dimethylaminoethyl methacrylate, dimethylaminoethyl acrylate, dimethylaminopropyl acrylate, diethylaminopropyl acrylate, dimethylaminobutyl acrylate, dimethylaminoethyl methacrylate, diethylaminoethyl methacrylate, dimethylaminoneopentyl acrylate and dimethylaminoneopentyl methacrylate.

Useful water-soluble polymers d) include polyvinyl alcohol, polyvinylpyrrolidone, starch, starch derivatives, polyethyleneimines, polyglycols, polymers formally constructed wholly or partly of vinylamine monomers, such as partially or completely hydrolyzed polyvinylamide (so-called "polyvinylamine") or polyacrylic acids, preferably polyvinyl alcohol and starch.

The polymerization is optionally carried out in the presence of customary polymerization regulators. Suitable polymerization regulators are for example thio compounds, such as thioglycolic acid, mercapto alcohols, for example 2-mercaptoethanol, mercaptopropanol and mercaptobutanol, dodecyl mercaptan, formic acid, ammonia and amines, for example ethanolamine, diethanolamine, triethanolamine, triethylamine, morpholine and piperidine.

The monomers (a), (b) and optionally (c) are (co)polymerized with each other, optionally in the presence of the water-soluble polymers d), in 20% to 80%, preferably 20% to 50% and especially 30% to 45% by weight aqueous solution in the presence of polymerization initiators. Useful polymerization initiators include all compounds that disintegrate into free radicals under the polymerization conditions, examples being peroxides, hydroperoxides, hydrogen peroxide, persulphates, azo compounds and the so-called redox initiators. The use of water-soluble initiators is preferred. It is advantageous in some cases to use mixtures of various polymerization initiators, examples being mixtures of hydrogen peroxide and sodium or potassium peroxodisulphate. Mixtures of hydrogen peroxide and sodium peroxodisulphate can be used in any desired ratio. Suitable organic peroxides are for example acetylacetone peroxide, methyl ethyl ketone peroxide, tert-butyl hydroperoxide, cumene hydroperoxide, tert-amyl perpivalate, tert-butyl perpivalate, tert-butyl perneohexanoate, tert-butyl perisobutyrate, tert-butyl per-2-ethylhexanoate, tert-butyl perisononanoate, tert-butyl permaleate, tert-butyl perbenzoate, tert-butyl per-3,5,5-trimethylhexanoate and tert-amyl perneodecanoate. Further suitable polymerization initiators are azo initiators, for example 2,2'-azobis(2-amidinopropane) dihydrochloride, 2,2'-azobis(N,N-dimethylene)-isobutyramidine dihydrochloride, 2-(carbamoylazo)isobutyronitrile and 4,4'-azobis(4-cyanovaleric acid). The polymerization initiators mentioned are used in customary amounts, for example in amounts of from 0.01 to 5 mol%, preferably 0.1 to 2 mol%, based on the monomers to be polymerized.

The redox initiators comprise, as oxidizing component, at least one of the above-indicated per compounds and a reducing component, for example ascorbic acid, glucose, sorbose, ammonium bisulfite, ammonium sulfite, ammonium thiosulphate, ammonium hyposulfite, ammonium pyrosulfite, ammonium sulfide, alkali metal bisulfite, alkali metal sulfite, alkali metal thiosulphate, alkali metal hyposulfite, alkali metal pyrosulfite, alkali metal sulfide, metal salts, such as iron(II) ions or silver ions, sodium hydroxymethylsulfoxylate, or sulfinic acid derivatives. The reducing component of the redox initiator is preferably ascorbic acid or sodium pyrosulfite. 1 · 10-5 to 1 mol% of the reducing component of the redox initiator and 1 · 10-5 to 5 mol% of the oxidizing component are used based on the amount of monomers used in the polymerization. Instead of the oxidizing component or in addition it is also possible to use one or more water-soluble azo initiators.

A redox initiator consisting of hydrogen peroxide, sodium peroxodisulphate and ascorbic acid is preferably used. These components are used for example in the concentrations of 1 · 10⁻² mol% of hydrogen peroxide, 0.084 mol% of sodium peroxodisulphate and 2.5 · 10-3 mol% of ascorbic acid, based on the monomers.

It is also possible to initiate the polymerization by the numerous other known means to initiate polymerizations. On example is initiating polymerization by irradiating with radiation of sufficiently high energy, in particular ultraviolet light. Usually, when initiating polymerization by ultraviolet light, compounds are added which decompose into radicals upon irradiation by ultraviolet light. Examples of such compounds are 2-hydroxi-2-methyl-1-phenyl-1-propanone and/or alpha,-alpha-dimethoxi-alpha-phenylacetophenone.

The aqueous monomer solution may comprise the initiator in dissolved or dispersed form. However, the initiators may also be added to the polymerization reactor separately from the monomer solution.

The preferred polymerization inhibitors require dissolved oxygen for optimum effect. Therefore, the polymerization inhibitors can be freed of dissolved oxygen prior to polymerization, by inertization, i.e., by flowing an inert gas, preferably nitrogen, through them. This is accomplished by means of inert gas, which can be introduced concurrently, countercurrently or at entry angles in between. Good commixing can be achieved for example with nozzles, static or dynamic mixers or bubble columns. The oxygen content of the monomer solution is preferably lowered to less than 1 weight ppm and more preferably to less than 0.5 weight ppm prior to polymerization. The monomer solution is optionally passed through the reactor using an inert gas stream.

The preparation of a suitable polymer as well as further suitable hydrophilic ethylenically unsaturated monomers a) are described for example in DE 199 41 423 A1, EP 686 650 A1, WO 01/45 758 A1 and WO 03/104 300 A1.

Superabsorbents are typically obtained by addition polymerization of an aqueous monomer solution and optionally a subsequent comminution of the hydrogel. Suitable methods of making are described in the literature. Superabsorbents are obtained for example by
- gel polymerization in the batch process or tubular reactor and subsequent comminution in meat grinder, extruder or kneader, as described for example in EP 445 619 A2 and DE 19 846 413 A1;
- polymerization in kneader with continuous comminution by contrarotatory stirring shafts for example, as described for example in WO 01/38 402 A1;
- polymerization on belt and subsequent comminution in meat grinder, extruder or kneader, as described for example in EP 955 086 A2, DE 38 25 366 A1 or US-6,241,928;
- emulsion polymerization, which produces bead polymers having a relatively narrow gel size distribution, as described for example in EP 457 660 A1;
- in situ polymerization on a woven fabric layer which, usually in a continuous operation, has previously been sprayed with aqueous monomer solution and subsequently been subjected to a photopolymerization, as described for example in WO 02/94 328 A2, WO 02/94 329 A1.

The cited references are expressly incorporated herein for details of process operation. The reaction is preferably carried out in a kneader or on a belt reactor.

Continuous gel polymerization is the economically preferred and therefore currently customary way of manufacturing superabsorbents. The process of continuous gel polymerization is carried out by first producing a monomer mixture by admixing the acrylic acid solution with the neutralizing agent, optional comonomers and/or further auxiliary materials at different times and/or locations and then transferring the mixture into the reactor or preparing the mixture as an initial charge in the reactor. The initiator system is added last to start the polymerization. The ensuing continuous process of polymerization involves a reaction to form a polymeric gel, i.e., a polymer swollen in the polymerization solvent - typically water - to form a gel, and the polymeric gel is already comminuted in the course of a stirred polymerization. The polymeric gel is subsequently dried, if necessary, and also chipped ground and sieved and is transferred for further surface treatment.

The acid groups of the hydrogels obtained are partially neutralized in an acid neutralization step, generally to an extent of at least 25 mol%, preferably to an extent of at least 50 mol% and more preferably at least 60 mol% and generally to an extent of not more than 85 mol%, preferably not more than 80 mol%, and more preferably not more than 75 mol%.

Neutralization can also be carried out after polymerization, at the hydrogel stage. But it is also possible to carry out the neutralization to the desired degree of neutralization wholly or partly prior to polymerization. In the case of partial neutralization and prior to polymerization, generally at least 10 mol%, preferably at least 15 mol% and also generally not more than 40 mol%, preferably not more than 30 mol% and more preferably not more than 25 mol% of the acid groups in the monomers used are neutralized prior to polymerization by adding a portion of the neutralizing agent to the monomer solution.

The desired final degree of neutralization is in this case only set toward the end or after the polymerization, preferably at the level of the hydrogel prior to its drying. The monomer solution is neutralized by admixing the neutralizing agent. The hydrogel can be mechanically comminuted in the course of the neutralization, for example by means of a meat grinder or comparable apparatus for comminuting gellike masses, in which case the neutralizing agent can be sprayed, sprinkled or poured on and then carefully mixed in. To this end, the gel mass obtained can be repeatedly meat-grindered for homogenization.

Neutralization of the monomer solution to the desired final degree of neutralization prior to polymerization by addition of the neutralizing agent or conducting the neutralization after polymerization is usually simpler than neutralization partly prior to and partly after polymerization and therefore is preferred.

The as-polymerized gels are optionally maintained for some time, for example for at least 30 minutes, preferably at least 60 minutes and more preferably at least 90 minutes and also generally not more than 12 hours, preferably for not more than 8 hours and more preferably for not more than 6 hours at a temperature of generally at least 50°C and preferably at least 70°C and also generally not more than 130°C and preferably not more than 100°C, which further improves their properties in many cases.

The neutralized hydrogel is then dried with a belt or drum dryer until the residual moisture content is preferably below 15% by weight and especially below 10% by weight, the water content being determined by EDANA (European Disposables and Nonwovens Association) recommended test method No. 430.2-02 "Moisture content". The dry superabsorbent consequently contains up to 15% by weight of moisture and preferably not more than 10% by weight. The decisive criterion for classification as "dry" is in particular a sufficient flowability for handling as a powder, for example for pneumatic conveying, pack filling, sieving or other processing steps involved in solids processing technology. Optionally, however, drying can also be carried out using a fluidized bed dryer or a heated ploughshare mixer. To obtain particularly colourless products, it is advantageous to dry this gel by ensuring rapid removal of the evaporating water. To this end, dryer temperature must be optimized, air feed and removal has to be policed, and at all times sufficient venting has to be ensured. Drying is naturally all the more simple - and the product all the more colourless - when the solids content of the gel is as high as possible. The solvent fraction at addition polymerization is therefore set such that the solid content of the gel prior to drying is therefore generally at least 20% by weight, preferably at least 25% by weight and more preferably at least 30% by weight and also generally not more than 90% by weight, preferably not more than 85% by weight and more preferably not more than 80% by weight. It is particularly advantageous to vent the dryer with nitrogen or some other nonoxidizing inert gas. Optionally, however, simply just the partial pressure of oxygen can be lowered during drying to prevent oxidative yellowing processes. But in general adequate venting and removal of the water vapour will likewise still lead to an acceptable product. A very short drying time is generally advantageous with regard to colour and product quality.

The dried hydrogel (which is no longer a gel (even though often still called that) but a dry polymer having superabsorbing properties, which comes within the term "superabsorbent") is preferably ground and sieved, useful grinding apparatus typically including roll mills, pin mills, hammer mills, cutting mills or swing mills. The particle size of the sieved, dry hydrogel is preferably below 1000 µm, more preferably below 900 µm and most preferably below 850 µm and preferably above 80 µm, more preferably above 90 µm and most preferably above 100 µm.

Very particular preference is given to a particle size (sieve cut) in the range from 106 to 850 µm. Particle size is determined according to EDANA (European Disposables and Nonwovens Association) recommended test method No. 420.2-02 "Particle size distribution".

The dry superabsorbing polymers thus produced are typically known as "base polymers" and are then surface postcrosslinked. Surface postcrosslinking can be accomplished in a conventional manner using dried, ground and classified polymeric particles. For surface postcrosslinking, compounds capable of reacting with the functional groups of the base polymer by crosslinking are applied, usually in the form of a solution, to the surface of the base polymer particles. Usually, a surface crosslinker solution is first applied to the base polymer by contacting base polymer and crosslinker solution, and then the formation of surface crosslinks is effected or completed by heat treatment. The contacting step leads to a coating of surface crosslinking solution on the base polymer particles, possibly to some crosslinking depending on reactivity of the crosslinker and temperature applied during the contacting step, and the heat treatment step to a finished surface-crosslinked superabsorbent.

The base polymer particles are surface crosslinked with a polyvalent metal salt solution comprising at least two different polyvalent metal salts, at least one of which comprises a chelate-forming anion. Preferably, the base polymers are also surface-crosslinked by at least one organic (post)crosslinker. Contacting the base polymer with a surface postcrosslinking solution (again, "surface crosslinking" is used synonymously) is typically carried out by spraying the surface postcrosslinking solution of the surface postcrosslinker ("surface crosslinker") onto the hydrogel or the dry base polymer powder. All crosslinkers may be applied in one solutions, but most often organic crosslinkers and polyvalent metal salts are applied in separate solutions to avoid solubility problems.

Suitable organic postcrosslinking agents are for example:
- di- or polyepoxides, for example di- or polyglycidyl compounds such as phosphonic acid diglycidyl ether, ethylene glycol diglycidyl ether, bischlorohydrin ethers of polyalkylene glycols,
- alkoxysilyl compounds,
- polyaziridines, compounds comprising aziridine units and based on polyethers or substituted hydrocarbons, for example bis-N-aziridinomethane,
- polyamines or polyamidoamines and also their reaction products with epichlorohydrin,
- polyols such as ethylene glycol, 1,2-propanediol, 1,4-butanediol, glycerol, methyltriglycol, polyethylene glycols having an average molecular weight Mw of 200 - 10 000, di- and polyglycerol, pentaerythritol, sorbitol, the ethoxylates of these polyols and also their esters with carboxylic acids or carbonic acid such as ethylene carbonate or propylene carbonate,
- carbonic acid derivatives such as urea, thiourea, guanidine, dicyandiamide, 2-oxazolidinone and its derivatives, bisoxazoline, polyoxazolines, di- and polyisocyanates,
- di- and poly-N-methylol compounds such as for example methylenebis(N-methylolmethacrylamide) or melamine-formaldehyde resins,
- compounds having two or more blocked isocyanate groups such as for example trimethylhexamethylene diisocyanate blocked with 2,2,3,6-tetramethylpiperidin-4-one.

If necessary, acidic catalysts can be added, examples being p-toluenesulfonic acid, phosphoric acid, boric acid or ammonium dihydrogenphosphate.

Particularly suitable postcrosslinking agents are di- or polyglycidyl compounds such as ethylene glycol diglycidyl ether, the reaction products of polyamidoamines with epichlorohydrin, 2-oxazolidinone and N-hydroxyethyl-2-oxazolidinone.

The solvent used for the surface postcrosslinker is a customary suitable solvent, examples being water, alcohols, DMF, DMSO and also mixtures thereof. Examples of suitable alcohols are monools, diols, triols or polyols, preferably of alcohols having one to eight carbon atoms. Preferred are the propanoles. Most preferably, the alcohol is selected from the group consisting of propylene glycol, 1,3-propandiol, 1-propanol, 2-propanol and mixtures thereof. Particular preference is given to water and water-alcohol mixtures, examples being water-methanol, water-1,2-propanediol, water-2-propanol and water-1,3-propanediol.

The spraying with a solution of the postcrosslinker is preferably carried out in mixers having moving mixing implements, such as screw mixers, paddle mixers, disk mixers, plowshare mixers and shovel mixers. Particular preference is given to vertical mixers and very particular preference to plowshare mixers and shovel mixers. Useful and known mixers include for example Lödige^{®}, Bepex^{®}, Nauta^{®}, Processall^{®} and Schugi^{®} mixers. Very particular preference is given to high speed mixers, for example of the Schugi-Flexomix^{®} or Turbolizer^{®} type.

The polymer is further surface crosslinked using a mixture prepared by dissolving at least two different polyvalent metal salts. Preferably, the polyvalent metal salts are water-soluble. Water-soluble polyvalent metal salts comprise bi- or more highly valent ("polyvalent") metal cations capable of reacting with the acid groups of the polymer to form complexes. Examples of polyvalent cations are metal cations such as Mg²⁺, Ca²⁺, Al³⁺, Sc³⁺, Ti⁴⁺, Mn²⁺, Fe^{2+/3+}, Co²⁺, Ni²⁺, Cu²⁺, Zn²⁺, Y³⁺, Zr⁴⁺, La³⁺, Ce⁴⁺, Hf⁴⁺, and Au³⁺. Preferred metal cations are Mg²⁺, Ca²⁺, Al³⁺, Ti⁴⁺, Zr⁴⁺ and La³⁺, and particularly preferred metal cations are Al³⁺, Ti⁴⁺ and Zr⁴⁺. Most preferred is Al³⁺. Of the metal cations mentioned, any metal salt can be used that has sufficient solubility in the solvent to be used.

One or more of the at least two polyvalent metal salts preferably is a polyvalent metal salts with a weakly complexing anion. Examples of such anions are chloride, nitrate and sulphate, hydrogen sulphate, carbonate, hydrogen carbonate, nitrate, phosphate, hydrogen phosphate, dihydrogen phosphate and carboxylate, such as acetate.

It is particularly preferred to use aluminium sulphate as one of the polyvalent metal salts.

At least one of the polyvalent metal salts used for surface crosslinking according to this invention comprises a chelate-forming anion. A chelate-forming anion is an anion capable of forming chelate complexes. A chelate complex is a complex where at least one of the ligands comprises more than one electron-donor moiety and thus occupies more than one ligand position on a central atom, thus forming a ring structure. Generally, chelate-forming anions are anions that comprise not only the functional group bearing the anion's negative charge, but also another electron-donor functional group. Particularly suitable functional groups comprise carboxylic groups, hydroxy groups, sulfonic acid groups, amino groups or thio groups.

Non-limiting preferred examples of chelate-forming anions suitable in this invention are the anions of organic diacids or hydroxyacids such as oxalic acid, glyoxylic acid, glycolic acid (hydroxiacetic acid), lactic acid, 3-hydroxypropionic acid, malonic acid, fumaric acid, maleic acid, succinic acid, tartaric acid, glutaric acid, adipic acid, sorbic acid, citric acid, ortho-, meta- or para-hydroxy benzoic acid, ortho-, meta or paramethoxiybenzoic acid or gluconic acid. Among these, lactate is particularly preferred.

It is particularly preferred to use aluminium lactate as polyvalent metal salt comprising a chelate-forming anion.

The superabsorbent is contacted with the polyvalent metal salt generally in the form of a polyvalent metal salt solution. Examples of suitable solvents are water, alcohols DMF, DMSO and also mixtures thereof. Examples of suitable alcohols are monools, diols, triols or polyols, preferably of alcohols having one to eight carbon atoms. Preferred are the propanoles. Most preferably, the alcohol is selected from the group consisting of propylene glycol, 1,3-propandiol, 1-propanol, 2-propanol and mixtures thereof. Particular preference is given to water and water-alcohol mixtures such as for example water-methanol, water-1,2-propanediol, water-2-propanol and water-1,3-propanediol.

The polyvalent metal salt solution is prepared by dissolving at least two polyvalent metal salts, one of which comprises a chelate-forming anion, in the solvent. Generally, it is sufficient to use a solution comprising two salts.

Contacting the superabsorbent polymer with solution of a polyvalent cation is carried out in the same way as that described above for organic surface postcrosslinker.

Preferably, the polyvalent metal salt solution is applied in the presence of an alcohol. It is possible, although not preferred because of stability concerns, to apply a stock solution containing organic crosslinker, polyvalent metal salts and alcohol as surface crosslinking agent. It is possible, however, and one preferred embodiment of the present invention, to mix a solution containing organic crosslinker with a solution of polyvalent metal salts, wherein at least one of these two solutions contains an alcohol, and if only one contains alcohol, preferably the solution of organic crosslinker contains it, directly in or shortly upstream of the nozzle used for spraying the surface crosslinking agent, so that the superabsorbent is treated with a mixture comprising organic crosslinker, polyvalent metal salt and alcohol. In another preferred embodiment of the invention, the superabsorbent is first treated with a solution containing an organic crosslinker and then with a solution containing the polyvalent metal salts. Again, at least one of these two solutions contains an alcohol, and if only one contains alcohol, it is preferably the solution containing the organic crosslinker.

Generally, the amount of organic crosslinker applied to the superabsorbent is at least 50 wt.-ppm, preferably at least 100 wt.-ppm, more preferably at least 200 wt.-ppm and generally not more than 1 wt.-%, preferably not more than 0,5 wt.-% and more preferably not more than 2000 wt.-ppm, based on the weight of the base polymer.

The total amount of polyvalent metal salts applied is generally at least 50 wt.-ppm, preferably at least 1000 wt.-ppm and more preferably at least 2000 wt.-ppm and generally not more than 5 wt.-%, preferably not more than 3 wt.-% and more preferably not more than 1 wt.-%, based on the weight of the base polymer. Typically, at least 10 wt.-ppm, preferably at least 200 wt.-ppm and more preferably at least 400 wt.-ppm and typically not more than 1 wt.-%, preferably not more than 0,6 wt.-% and more preferably not more than 0.2 wt.-%, based on base polymer, are polyvalent metal salt comprising a chelate-forming anion. In turn, generally at least 40 wt.-ppm, preferably at least 800 wt.-ppm and more preferably at least 1600 wt.-ppm and generally not more than 4 wt.-%, preferably not more than 2.4 wt.-% and more preferably not more than 0.8 wt.-%, based on base polymer, are another polyvalent metal salt.

If an alcohol is part of the polyvalent metal salts solvent, its amount is generally at least 1000 wt.-ppm, preferably at least 2000 wt.-ppm and more preferably at least 3000 wt.-% and generally not more than 15 wt.-%, preferably not more than 10 wt.-% and more preferably not more than 5 wt.-%, based on the weight of base polymer.

The step of contacting a superabsorbent base polymer with an organic crosslinker and a polyvalent metal salt solution in the presence of an alcohol is followed by a thermal treatment step, essentially to effect the surface-postcrosslinking reaction (yet usually just referred to as "drying"), preferably in a downstream heated mixer ("dryer") at a temperature of generally at least 50°C, preferably at least 80°C and more preferably at least 80°C and also generally not more than 300°C, preferably not more than 250°C and more preferably not more than 200°C. The average residence time (i.e., the averaged residence time of the individual particles of superabsorbent) in the dryer of the superabsorbent to be treated is generally at least 1 minute, preferably at least 3 minutes and more preferably at least 5 minutes and also generally not more than 6 hours, preferably 2 hours and more preferably not more than 1 hour. As well as the actual drying taking place, not only any products of scissioning present but also solvent fractions are removed. Thermal drying is carried out in customary dryers such as tray dryers, rotary tube ovens or heatable screws, preferably in contact dryers. Preference is given to the use of dryers in which the product is agitated, i.e., heated mixers, more preferably shovel dryers and most preferably disk dryers. Bepex^{®} dryers and Nara^{®} dryers are suitable dryers for example. Fluidized bed dryers can also be used. But drying can also take place in the mixer itself, by heating the jacket or blowing a preheated gas such as air into it. But it is also possible for example to utilize an azeotropic distillation as a drying process. The crosslinking reaction can take place not only before but also during drying.

When water is present in the base polymer or organic crosslinker solution, it is preferred to conduct the heat treatment at conditions sufficient to reduce the moisture content of the resulting surface-crosslinked superabsorbent to a value of less than 1 wt.-%, based on the total amount of surface-crosslinked superabsorbent.

In a preferred embodiment of this invention, the surface-crosslinked superabsorbent is then re-moisturised by adding water.

Methods for adding liquids such as water to particulate solids such as superabsorbents are known, and any known method may be used. In all cases, water is preferably added while the superabsorbent is gently agitated in a mixer to achieve a homogeneous distribution of moisture in the superabsorbent. There are two particularly convenient ways to add water to superabsorbents. One is to provide a separate re-moisturizing mixer and the other is to add water in a cooler employed for cooling the product after the heat-treatment that finalizes the surface postcrosslinking.

Mixers suitable for re-moisturising superabsorbents are known. The type of mixer to be used is not particularly limited, for example single or double screw mixers, paddle mixers, vortex mixers, rolling mixers, ribbon mixers, air mixers, tumbling mixers and fluidized beds, or any other apparatus that allows homogeneous mixing of particulate solids with liquids. Preference is given to the use of continuously operating paddle mixers, for example the paddle mixers obtainable from Nara Machinery Co., Ltd., Tokyo, Japan, the Turbulizer^{®} obtainable from Bepex International LLC, Minneapolis, U.S.A. or from Hosokawa Micron BV, Doetinchem, The Netherlands, or the paddle mixers obtainable from Gebr. Ruberg GmbH & Co. KG, Nieheim, Germany.

Water can be added in any way and is preferably added by spraying. Water may be added using one or more nozzles, It is preferred to use an even number of nozzles. Any nozzle suitable to spray water can be used. When using the preferred continuous paddle mixer, the superabsorbent is gradually advanced along the shaft(s) (a single-shaft-mixer is preferred), and water is added through one or more nozzles generally located at a position corresponding to at least 0 %, preferably at least 30 and generally not more than 90 %, preferably not more than 70 % of the average residence time of superabsorbent in the cooler/mixer. If more than one nozzles are used, water may be added at several places in the mixer, such as for instance one nozzle or set of nozzles located at a position or positions corresponding to the range of 20 % to 40 %, another one or set of located at a position or positions corresponding to the range of 60 to 80 % of the average residence time.

The temperature at which water is added typically is in the range of 40 to 95 °C. The average residence time of superabsorbent in there-moisturizing step typically is in the range of 5 to 120 minutes.

Generally, the amount of water added is sufficient to achieve a moisture content of the re-moisturized superabsorbent of at least 1 wt.-%, preferably at least 3 wt.-% and generally not more than 7 wt.-%, preferably not more than 5 wt.-%, based on the total weight of re-moisturized superabsorbent.

Water may also be added in a cooling step employed for cooling the product after the heat-treatment that finalizes the surface postcrosslinking.

After any drying or heat treatment step, it is advantageous but not absolutely necessary to cool the product after drying. Cooling can be carried out continuously or discontinuously, conveniently by conveying the product continuously into a cooler downstream of the dryer. Any apparatus known for removing heat from pulverulent solids can be used, in particular any apparatus mentioned above as a drying apparatus, provided it is supplied not with a heating medium but with a cooling medium such as for example with cooling water, so that heat is not introduced into the superabsorbent via the walls and, depending on the design, also via the stirrer elements or other heat-exchanging surfaces, but removed from the superabsorbent. Preference is given to the use of coolers in which the product is agitated, i.e., cooled mixers, for example shovel coolers, disk coolers or paddle coolers, for example Nara^{®} or Bepex^{®} coolers. The superabsorbent can also be cooled in a fluidized bed by blowing a cooled gas such as cold air into it. The cooling conditions are set such that a superabsorbent having the temperature desired for further processing is obtained. Typically, the average residence time in the cooler will be in general at least 1 minute, preferably at least 3 minutes and more preferably at least 5 minutes and also in general not more than 6 hours, preferably 2 hours and more preferably not more than 1 hour, and cooling performance will be determined such that the product obtained has a temperature of generally at least 0°C, preferably at least 10°C and more preferably at least 20°C and also generally not more than 100°C, preferably not more than 80°C and more preferably not more than 60°C.

In a preferred embodiment of the present invention, re-moisturizing is done during a cooling step after surface postcrosslinking. The mixing apparatus described above usually is equipped with heat transfer equipment and can directly be used as coolers. In that case, the process conditions described for the re-moisturizing step may be used for the cooling step as well, although it is be possible to use one part of the same mixer/cooler for re-moisturizing and another for cooling at different process conditions, depending on the particular apparatus used. In any case, water preferably is added only at positions in the cooler where the superabsorbent temperature is below 100 °C.

Optionally, the superabsorbent is provided with further customary additives and auxiliary materials to influence storage or handling properties. Examples thereof are permeability enhancing agents other than surface crosslinkers, such as particulate solids (silica is widely used) or cationic polymers to further enhance permeability, colorations, opaque additions to improve the visibility of swollen gel, which is desirable in some applications, surfactants, cohesion control agents to improve flowability or the like. These additives and auxiliary materials can each be added in separate processing steps by methods generally known in the art, but one convenient method may be to add them to the superabsorbent in the cooler, for example by spraying the superabsorbent with a solution or adding them in finely divided solid or in liquid form, if this cooler provides sufficient mixing quality. It is possible and may be rather convenient to add such additives and auxiliaries partly or totally together with the water in the re-moisturizing step, preferably a combined re-moisturizing and cooling step in a cooler/mixer after surface postcrosslinking as described above. One convenient method for adding particulate solids in a cooler/mixer may be spraying with water in two-or multi-component nozzles. Generally, however, it is preferred to re-moisturise the superabsorbent with pure water.

The final surface-crosslinked superabsorbent is optionally ground and/or sieved in a conventional manner. Grinding is typically not necessary, but the sieving out of agglomerates which are formed or undersize is usually advisable to set the desired particle size distribution for the product. Agglomerates and undersize are either discarded or preferably returned into the process in a conventional manner and at a suitable point; agglomerates after comminution. The superabsorbent particle size is preferably not more than 1000 µm, more preferably not more than 900 µm, most preferably not more than 850 µm, and preferably at least 80 µm, more preferably at least 90 µm and most preferably at least 100 µm. Typical sieve cuts are for example 106 to 850 µm or 150 to 850 µm.

### Superabsorbent Property Test Methods

### Centrifuge Retention Capacity (CRC)

The method for determination of the Centrifuge Retention Capacity (CRC) is described in US patent application no. US 2002/0 165 288 A1, paragraphs [0105] and [0106].

### Absorption Under Load 0.9 psi (AUL 0.9 psi)

The procedure for determining AUL 0.9 psi is disclosed in WO 00/62 825 , pages 22 - 23 (referred to as "Absorbency Under Load" therein). A 317 gram weight is used to obtain the AUL 0.9 psi value.

### Particle Size Distribution (PSD)

Particle Size Distribution is determined using EDANA (European Disposbles and Nonwovens Association, Avenue Eugène Plasky, 157, 1030 Brussels, Belgium, www.edana.org) Test Method 420.2-02 (available from EDANA).

### Residual Moisture Content

The method for determination of the Residual Moisture Content is described in WO 01/25 290 A1, page 19, line 24 through page 20, line 8.

### Free Swell Gel Bed Permeability (Free Swell GBP)

The method for determination of the Free Swell Gel Bed Permeability (Free Swell GBP) is described in US patent application no. US 2005/0 256 757 A1, paragraphs [0061] through [0075].

### Examples

### Example 1 (comparative)

A base polymer was produced using the process described in WO 01/38 402 A1 by copolymerising a mixture of 25 mole-% acrylic acid and 75 mole-% sodium acrylate with an internal crosslinker. This polymer was designated polymer A.

### Example 2 (comparative):

1 kg of the polymer A powder was put into a mixer (laboratory ploughshare mixer model M 5, manufactured by Gebrüder Lödige Maschinenbau GmbH, Paderborn, Germany). A first surface crosslinking solution was prepared by mixing 0.95 g of Denacol^{®} EX 810 (ethylene glycol diglycidyl ether, obtained from Nagase ChemteX Corporation, Osaka, Japan), 6.0 g of propylene glycol, and 7.5 g of deionized water. As second surface crosslinking solution, 111 g of a 27 wt.-% aqueous aluminum sulfate solution were used. At a mixer speed of 449 rpm, the surface crosslinker solutions were added dropwise using a syringe to the polymer powder over a three minute time period. The mixer was then stopped, product sticking to the wall of the mixing vessel was scraped off (and re-united with the bulk), and mixing was continued for two more minutes at 449 rpm. The batch was then discharged into two stainless steel pans and placed in an oven at 180 °C for one hour. The pans the were removed from the oven and allowed to cool in a desiccator. The cooled product was then sifted, and the 850-150 micron cut designated Polymer B.

### Example 3

Example 2 was repeated, however, 1 g of aluminium lactate was added to the aluminium sulphate solution. The resulting polymer was designated Polymer C.

### Example 4

Example 2 was repeated, however, 2 g of aluminium lactate were added to the aluminium sulphate solution. The resulting polymer was designated Polymer D

The properties of the polymers thus obtained are summarised in table 1.

**Table 1**

| Polymer | CRC [g/g] | AUL 09.psi [g/g] | Free Swell GBP [Darcies] |
|---|---|---|---|
| A | 38.3 | - | - |
| B | 33.0 | 17.0 | 92.1 |
| C | 34.3 | 19.4 | 59.5 |
| D | 35.0 | 19.2 | 40.7 |

Rather surprisingly, the examples show that the lactate additions enhances the AUL value without affecting the CRC value. In standard superabsorbents, these values typically are inversely related. The Free Swell GBP is not overly diminished and remains in the range typically desired for surface-crosslinked superabsorbents.

## Claims

1. A process for producing a surface-crosslinked superabsorbent that comprises the steps of contacting a superabsorbent base polymer with an polyvalent metal salt solution produced by dissolving at least two different polyvalent metal salts, at least one of which comprises a chelate-forming anion, and heat-treating to produce a surface-crosslinked superabsorbent.

2. The process of claim 1, wherein the base polymer is contacted with a total amount of 50 wt.-ppm to 5 wt.-%, based on base polymer, polyvalent metal salts.

3. The process of claims 1 or 2, wherein the base polymer is also contacted with an organic surface crosslinker.

4. The process of any of claims 1 to 3, wherein the polyvalent metal salts comprise a polyvalent metal cation selected from the group consisting of Al³⁺, Ti⁴⁺ and Zr⁴⁺.

5. The process of claim 4, wherein the polyvalent metal cation is Al³⁺.

6. The process of any of claims 1 to 5, wherein the chelate-forming anion is a hydroxy carboxylic acid anion.

7. The process of claim 6, wherein the chelate-forming anion is lactate.

8. The process of claim 7, wherein the polyvalent metal salt solution is produced by dissolving aluminium sulphate and aluminium lactate in a water-containing solvent.

9. The process of claim 8, wherein the polyvalent metal salt solution is produced by dissolving 40 wt.-ppm to 4 wt.-%, based on base polymer, aluminium sulphate and 10 wt.-ppm to 1 wt.-%, based on base polymer, aluminium lactate in a water-containing solvent.

10. The process of any of claims 1 to 9, wherein the surface-crosslinked superabsorbent is re-moisturised to a moisture content of 1 to 7 wt.-% based on the total weight of superabsorbent.

## Patentansprüche

1. Verfahren zur Herstellung eines oberflächenvernetzten Superabsorbers, umfassend die Schritte des Inberührungbringens eines Superabsorber-Grundpolymers mit einer durch Lösen von mindestens zwei verschiedenen mehrwertigen Metallsalzen, von denen mindestens eines ein Chelat bildendes Anion umfasst, hergestellten Lösung von mehrwertigem Metallsalz und des Wärmebehandelns zur Bildung eines oberflächenvernetzten Superabsorbers.

2. Verfahren nach Anspruch 1, bei dem das Grundpolymer mit einer Gesamtmenge von 50 Gew.-ppm bis 5 Gew.-%, bezogen auf das Grundpolymer, mehrwertiger Metallsalze in Berührung gebracht wird.

3. Verfahren nach Anspruch 1 oder 2, wobei das Grundpolymer auch mit einem organischen Oberflächenvernetzer in Berührung gebracht wird.

4. Verfahren nach einem der Ansprüche 1 bis 3, bei dem die mehrwertigen Metallsalze ein mehrwertiges Metallkation aus der Gruppe bestehend aus Al³⁺, Ti⁴⁺ und Zr⁴⁺ umfassen.

5. Verfahren nach Anspruch 4, bei dem es sich bei dem mehrwertigen Metallkation um Al³⁺ handelt.

6. Verfahren nach einem der Ansprüche 1 bis 5, bei dem es sich bei dem chelatbildenden Anion um ein Hydroxycarbonsäure-Anion handelt.

7. Verfahren nach Anspruch 6, bei dem es sich bei dem chelatbildenden Anion um Lactat handelt.

8. Verfahren nach Anspruch 7, bei dem die Lösung von mehrwertigem Metallsalz durch Lösen von Aluminiumsulfat und Aluminiumlactat in einem wasserhaltigen Lösungsmittel hergestellt wird.

9. Verfahren nach Anspruch 8, bei dem die Lösung von mehrwertigem Metallsalz durch Lösen von 40 Gew.-ppm bis 4 Gel.-%, bezogen auf das Grundpolymer, Aluminiumsulfat und 10 Gew.-ppm bis 1 Gew.-%, bezogen auf das Grundpolymer, Aluminiumlactat in einem wasserhaltigen Lösungsmittel hergestellt wird.

10. Verfahren nach einem der Ansprüche 1 bis 9, bei dem der oberflächenvernetzte Superabsorber bis zu einem Feuchtigkeitsgehalt von 1 bis 7 Gew.-%, bezogen auf das Gesamtgewicht des Superabsorbers, nachbefeuchtet wird.

## Revendications

1. Procédé de fabrication d'un superabsorbant réticulé en surface, comprenant les étapes qui consistent à mettre un polymère de base superabsorbant en contact avec une solution de sels de métaux polyvalents produite par dissolution d'au moins deux différents sels de métaux polyvalents, au moins un desdits sels de métaux polyvalents comprenant un anion formant un chélate, et à réaliser un traitement thermique pour produire un superabsorbant réticulé en surface.

2. Procédé selon la revendication 1, dans lequel le polymère de base est mis en contact avec une quantité totale de 50 ppm en poids à 5 % en poids, relativement au polymère de base, de sels de métaux polyvalents.

3. Procédé selon les revendications 1 ou 2, dans lequel le polymère de base est aussi mis en contact avec un agent de réticulation de surface organique.

4. Procédé selon l'une quelconque des revendications 1 à 3, dans lequel les sels de métaux polyvalents comprennent un cation de métal polyvalent sélectionné dans le groupe constitué de Al³⁺, de Ti⁴⁺ et de Zr⁴⁺.

5. Procédé selon la revendication 4, dans lequel le cation de métal polyvalent est Al³⁺.

6. Procédé selon l'une quelconque des revendications 1 à 5, dans lequel l'anion formant un chélate est un anion d'acide hydroxycarboxylique.

7. Procédé selon la revendication 6, dans lequel l'anion formant un chélate est un lactate.

8. Procédé selon la revendication 7, dans lequel la solution de sels de métaux polyvalents est produite par dissolution de sulfate d'aluminium et de lactate d'aluminium dans un solvant contenant de l'eau.

9. Procédé selon la revendication 8, dans lequel la solution de sels de métaux polyvalents est produite par dissolution de 40 ppm en poids à 4 % en poids, relativement au polymère de base, de sulfate d'aluminium, et de 10 ppm en poids à 1 % en poids, relativement au polymère de base, de lactate d'aluminium dans un solvant contenant de l'eau.

10. Procédé selon l'une quelconque des revendications 1 à 9, dans lequel le superabsorbant réticulé en surface est ré-humidifié jusqu'à une teneur en humidité de 1 % à 7 % en poids relativement au poids total de superabsorbant.
